(19) Europäisches Patentamt
European Patent Office
Office européen des brevets

(11) Publication number: 0 400 611 A2

# (12) EUROPEAN PATENT APPLICATION

(21) Application number: 90110271.5

(22) Date of filing: 30.05.90

(51) Int. Cl.[5]: C07C 229/60, C07K 5/06, C07K 5/08, C12Q 1/37

(30) Priority: 31.05.89 JP 137748/89

(43) Date of publication of application: 05.12.90 Bulletin 90/49

(84) Designated Contracting States: CH DE LI

(71) Applicant: NITTO BOSEKI CO., LTD.
1, Aza Higashi Gonome
Fukushima-shi(JP)

(72) Inventor: Kuroiwa, Katsumasa
1-44 Shimokitaimae, Asakamachi Arai
Koriyama-shi(JP)
Inventor: Nagasawa, Takeshi
19-10, Ryoke-7-chome
Urawa-shi(JP)
Inventor: Katayama, Katsuhiro
80-21, Daijuuchi, Tomitamachi
Koriyama-shi(JP)
Inventor: Nakatsuyama, Shuichi
198, Ohara, Fukuyamamachi Kubota
Koriyama-shi(JP)
Inventor: Matsuura, Hitoshi
38, Machiura, Hiwadamachi Takakura
Koriyama-shi(JP)

(74) Representative: Hansen, Bernd, Dr. Dipl.-Chem. et al
Hoffmann, Eitle & Partner Patent- und Rechtsanwälte Arabellastrasse 4
D-8000 München 81(DE)

## (54) Aminoacetophenone derivatives and method for determination of enzyme activity using the same.

(57) Novel aminoacetophenone derivatives represented by leucyl-4-acetylanilide are useful as synthetic substrates which provide high sensitivity and high specificity without being affected by interferrants in serum or plasma, such as bilirubin or hemoglobin. The aminoacetophenone derivatives are also useful as the starting materials of synthetic substrates. The aminoacetophenone derivatives are suitable as synthetic substrates used for the method for determination of a peptidase activity or a protease activity, especially in automated analysis equipments.

EP 0 400 611 A2

# AMINOACETOPHENONE DERIVATIVES AND METHOD FOR DETERMINATION OF ENZYME ACTIVITY USING THE SAME

## BACKGROUND OF THE INVENTION

### Field of the Invention

The present invention relates to novel aminoacetophenone derivatives and a method for determination of enzyme activity in serum or plasma using such derivatives as substrate.

### Related Art Statement

Where protease activity or peptidase activity is determined using synthetic substrates, synthetic substrates obtained by introducing a chromogenic group, etc. in amino acids or peptide derivatives have been widely used from old. Methods for determination of the enzyme activity using synthetic substrates include the following:

(1) Method using p-nitroaniline derivatives [Klin. Wochenschr., 45, 474 (1967), Clin. Chim. Acta, 7, 755 (1962), Japanese Patent Application Laid-open Nos. 52-3494 and 52-24590];

(2) Method using aniline derivatives [Japanese Patent Application Laid open Nos. 52-146693 and 52-52691];

(3) Method using naphthylamine derivatives [Cancer, 11, 283 (1958), Clin. Chim. Acta, 7, 755 (1962)];

(4) Method using coumarine derivatives [J. Biochem., 82, 1495 (1977)].

The methods using these synthetic substrates include either measuring absorbance or fluorescent intensity of a chromogenic group released from amino acids or peptide derivatives upon enzyme reaction, or by measuring absorbance of a dye produced upon a color-forming reaction of the released chromogenic group.

(1) According to the method using p-nitroaniline derivatives, it is possible to perform the initial velocity analysis in which a formation rate of a color produced by enzyme reaction can be directly measured. The method is thus advantageous in that an automated analysis measurement equipment is easily applicable to the method and a large number of samples can be handled, etc. However, the wavelength used for the measurement is between 400 and 415 nm so that the method is unavoidably affected by serum or plasma components, especially hemoglobin or bilirubin, leading to causes for an error in measurement data.

(2) The method using aniline derivatives comprises producing a dye from the chromogenic group formed upon enzyme reaction either by chemical reaction or using enzyme and, measuring its absorbance. According to this method, the measurement can be made at such a wavelength that is hardly affected by serum or plasma components. However, the operation is complicated and it is difficult to perform the initial velocity method so that it is difficult to apply this method to an automated analysis equipment which enables to handle a large number of samples. Turning to the method for producing a dye using enzyme, there are problems that enzyme reagents are very expensive, enzyme has poor stability, etc.

(3) The method using naphthylamine derivatives encounters the same problems as described in (2) above. In addition, carcinogenesis of naphthylamine derivatives has come into question. Therefore, the method is defective from a practical viewpoint.

(4) According to the method using coumarine derivatives, a fluorescent substance produced upon enzyme reaction is measured. The method is advantageous in that its sensitivity is extremely good but a fluorescent photometer is required for the measurement; the photometer is generally expensive and has not yet been mounted to an automated analysis equipment and hence, its application is restricted.

## SUMMARY OF THE INVENTION

Therefore, an object of the present invention is to provide novel aminoacetophenone derivatives which provide high sensitivity and high specificity, are useful as synthetic substrates enabling to measure with an automated analysis equipment by the initial velocity method without being affected by the coexisting components upon measurement and are also useful as starting materials for synthesis of synthetic substrates.

Another object of the present invention is to provide a method for determination of an enzyme activity using the aminoacetophenone derivative as synthetic substrate.

As a result of extensive investigations to solve the defects in the prior art methods, the present inventors have come to reach the present invention. That is, investigations have been made to synthesize novel aminoacetophenone derivatives and to develop a method for determining a peptidase activity and a protease activity by U.V. method, using these compounds as substrates. As a result, it has been found that such aminoacetophenone derivatives are advantageous in various aspects that the aminoacetophenone derivatives have made it possible to use a wavelength in the region of from about 335 to about 355 nm for the measurement so that a peptidase activity or a protease activity in serum and plasma can be determined accurately with good reproducibility.

That is, the present invention relates to aminoacetophenone derivatives represented by general formula (I):

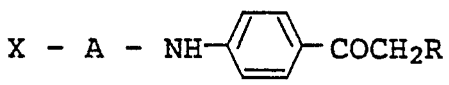

(I)

wherein R represents hydrogen, an alkyl group, hydroxy group or acetoxy group; X represents hydrogen atom, a group for irreversibly masking the terminal amino group or an amino-protective group conventionally used in peptide chemistry; A represents an L- or D-amino acid residue selected from the group consisting of phenylalanine, leucine, isoleucine, glutamic acid, pyroglytamic acid, serine, valine, arginine, lysine, proline, pipecolic acid, alanine and phenylalanine or a homologue thereof, or a residue of di- or tripeptide composed of 2 or 3 of these amino acids or a homologue thereof; provided that $\epsilon$-amino group of the lysine residue may optionally be protected with a protective group; and their acid addition salts.

The present invention also relates to a method for determination of a peptidase activity or a protease activity which comprises using the aminoacetophenone derivatives represented by general formula (I) described above.

## BRIEF DESCRIPTION OF THE DRAWINGS

Fig. 1 shows U.V. spectra of (a) p-aminoacetophenone (concentration, 50 $\mu$M) and (b) leucyl-4-acetylanilide (concentration, 50 $\mu$M), respectively, in 125 mM Tris buffer showing pH of 7.8 (25$^\circ$ C).

Fig. 2 shows (a) reaction time course and (b) reagent blank, in terms of LAP activity in serum when leucyl-4-acetylanilide is used as substrate.

Fig. 3 shows (a) reaction time course and (b) reagent blank, in terms of $\gamma$-GTP activity in serum when $\gamma$-glutamyl-4-acetylanilide is used as substrate.

Fig. 4 shows (a) reaction time course and (b) reagent blank, in terms of $\gamma$-GTP activity in serum when $\gamma$-glutamyl-4-($\omega$-acetoxyacetyl)anilide is used as substrate.

Fig. 5 shows (a) reaction time course and (b) reagent blank, in terms of $\gamma$-GTP activity in serum when $\gamma$-glutamyl-4-($\omega$-oxyacetyl)anilide is used as substrate.

Fig. 6 is a graph showing a standard curve for p-aminoacetophenone.

Fig. 7 is a graph showing a calibration curve prepared by measuring AT-III activity using a compound of Example 8 as substrate. The axis of ordinates indicates change in absorbance at 340 nm per minute and the axis of abscissas indicates AT-III activity.

Fig. 8 is a graph showing a calibration curve prepared by measuring PLG activity using a compound of Example 14 as substrate. The axis of ordinates indicates change in absorbance at 340 nm per minute and the axis of abscissas indicates PLG activity.

## DETAILED DESCRIPTION OF THE PREFERRED EMBODIMENTS

In the formula (I) described above, examples of R include an alkyl group having 1 to 5 carbon atoms such as methyl, ethyl, propyl, butyl, pentyl, etc. As the group shown by X for irreversibly masking the terminal amino group, for example, benzoyl group or the like can be exemplified. Examples of the amino-protective group ordinarily used in peptide chemistry include t-butyl-oxycarbonyl, benzyloxycarbonyl, p-methoxybenzyloxy-carbonyl, 9-fluorenylmethyloxycarbonyl, etc. Examples of the homologue of amino acid residue shown by A include a glutamic acid derivative residue having bound to the $\gamma$-carboxyl group of

glutamic acid a cycloalkyl group having 3 to 6 carbon atoms such as cyclopropyl, cyclobutyl, cyclopentyl, cyclohexyl, etc.; a serine derivative residue having bound to the β-hydroxy group of serine benzyl group, etc. Examples of the homologue of di- or tripeptide residue include a di-or tripeptide residue containing these amino acid derivative residues. Examples of the protective group for ε-amino group of the lysine residue include the aforesaid protective groups.

The aminoacetophenone derivatives of general formula (I) may also be in the form of acid addition salts thereof. Examples of such acid addition salts are inorganic acid salts such as hydrochlorides, hydrobromides, sulfates, phosphates, etc.; or organic acid salts such as succinates, citrates, malates, etc.

The aminoacetophenone derivatives represented by general formula (I) and their acid addition salts can be synthesized in a conventional manner principally used in peptide chemistry.

That is, the aminoacetophenone derivatives can be synthesized either by condensing compounds represented by the following formula (II):

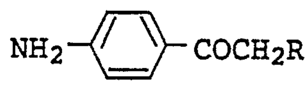

(II)

wherein R has the same significance as defined in general formula (I), with an appropriate amino acid, dipeptide or tripeptide, or by condensing the compounds of formula (II) stepwise with amino acids. In general, these condensation reactions can be carried out by protecting the amino group of the amino acid, dipeptide or tripeptide with a protective group as described above and activating the carboxyl group. The activation of the carboxyl group is performed by, for example, the carbodiimide method, the azide method, the acid anhydride method or the activated ester method. The condensation can be effected in a solvent ordinarily used, for example, dimethylformamide, pyridine, chloroform, methylene chloride, ethyl acetate, tetrahydrofuran, dioxan, etc.

The protective group is removed preferably at the end point of synthesis. The protective group can be removed generally by acid decomposition using hydrochloric acid/acetic acid, methanesulfonic acid/acetic acid, 25% hydrobromic acid/acetic acid, formic acid, hydrochloric acid/formic acid, hydrochloric acid/dioxan, hydrochloric acid/ethyl acetate, trifluoroacetic acid, etc.

4-Amino-ω-oxyacetophenone wherein R is hydroxy and 4-amino-ω-acetoxyacetophenone wherein R is acetoxy, in formula (II), can be prepared by known process (Beilstein, XIV, p. 49). Compounds wherein R is an alkyl group can also be prepared in a conventional manner.

Next, the method for determination of a peptidase activity or a protease activity in accordance with the present invention is explained below.

The peptidase activity or protease activity can be determined by mixing a sample containing peptidase or protease to be determined with the aminoacetophenone derivative of general formula (I) to conduct enzyme reaction and measuring an absorbance of the compound of formula (II) released, whereby the released compound of formula (II) is quantitatively determined.

In the case of using, for example, leucyl-4-acetylanilide, as the aminoacetophenone derivative of general formula (I), a sample containing peptidase or protease is mixed with leucyl-4-acetylanilide to perform enzyme reaction, whereby p-aminoacetophenone is released. In Fig. 1, U.V. spectra of p-aminoacetophenone (a) and leucyl-4-acetylanilide (b) are shown. p-Aminoacetophenone shows an absorption between about 335 and about 355 nm in a buffer solution but contrary to p-aminoacetophenone, leucyl-4-acetylanilide which is a synthetic substrate obtained by peptide binding shows an absorption in a wavelength region shorter than about 335 nm. Accordingly, the reaction can be traced in the measurement wavelength of from 335 to 355 nm by the U.V. method. In this case, interference with other components in serum or plasma can be reduced in the region around this wavelength. Accordingly, an increase in U.V. absorption of p-aminoacetophenone released from the substrate can be accurately traced, resulting in an extremely minimized error in measurement data.

By the use of the aminoacetophenone derivative of the present invention shown by general formula (I), the activity of peptidase or protease, for example, leucine aminopeptidase, γ-glutamyltranspeptidase, plasminogen, antithrombin, etc. can be determined. Examples of the aminoacetophenone derivative of the present invention which are preferred as the substrate include substrates of Examples 1, 2, 3, 4, 6, 8 and 14 and the following tripeptide substrates.

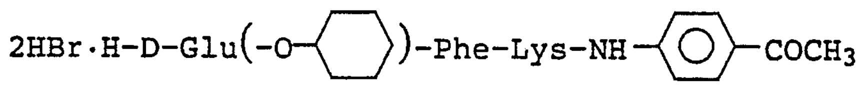

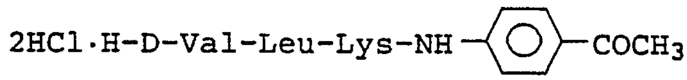

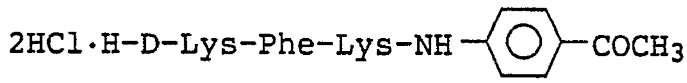

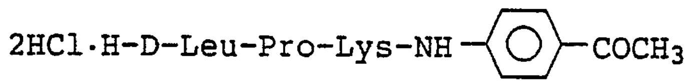

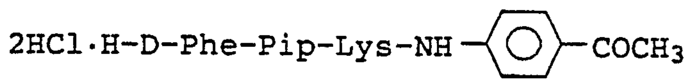

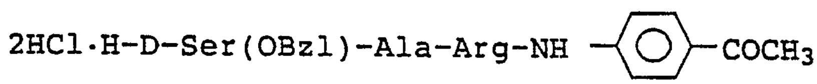

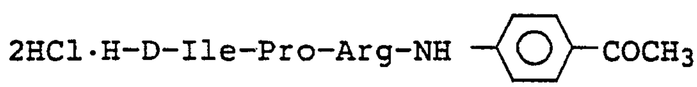

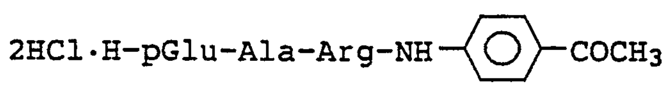

According to the present invention, the new aminoacetophenone derivatives are provided; using these compounds as substrates for determination of enzyme activity, peptidase or protease can be measured at a wavelength region of from 335 to 355 nm in which measurement data are hardly affected by hemoglobin or bilirubin, etc. present in serum or plasma. It is also possible to determine the enzyme activity by the initial velocity method so that it is unnecessary to take a blank for every sample, resulting in rapid measurement and handling a large number of samples. In addition, such a method is readily applicable to an automated analysis equipment widely spread in medical facilities like hospitals, etc.

As described above, the method for determination of a peptidase activity or a protease activity using the novel aminoacetophenone derivatives of the present invention has solved the problems encountered in the prior art. The method of the present invention is also characterized by various advantages and characteristics. Therefore, the method of the present invention is extremely useful for determining the peptidase activity or protease activity in daily clinical inspections.

Hereafter the present invention is described in more detail with reference to the examples below but is not deemed to be limited thereto.

BOC : t-butyloxycarbonyl
Z : benzyloxycarbonyl
OBut : t-butyl ester
Ac: acetyl
AcOEt : ethyl acetate
Leu : leucine
Glu : glutamic acid
Arg : arginine
Phe : phenylalanine
Pro : proline

Lys : lysine
AcOH : acetic acid
WSCD : water-soluble carbodiimide
n-Hex : n-hexane
TsOH : p-toluenesulfonic acid
SDP : 2-mercapto-4,6-dimethylpyrimidine
HOBt : 1-hydroxybenzotriazole
NEM : N-ethylmorpholine
MeOH : methanol
OSu N-hydroxysuccinimide ester
n-BuOH : n-butanol

Example 1

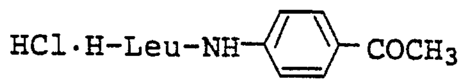

After 8.1 g of BOC-Leu-OH and 4.7 g of p-aminoacetophenone were dissolved in 70 ml of pyridine, the solution was cooled to 0° C. While stirring, 8.1 g of WSCD was added to the solution and the mixture was reacted at 0° C for 4 hours, and then at room temperature overnight. After completion of the reaction, 300 ml of AcOEt was added to the reaction solution. The mixture was washed with cold 5% HCl, saturated $NaHCO_3$ aqueous solution and then saturated sodium chloride aqueous solution. The organic phase was then dried over anhydrous magnesium sulfate. The solvent was distilled off in vacuum to give the crude product. The crude product was subjected to silica gel chromatography (Merck Art 7734, AcOEt : n-Hex = 1 : 4). The desired fractions were collected and concentrated to give 11.4 g of:

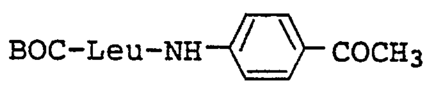

as an oily substance. In a moistureproof state, 77 ml of 2N-HCl/AcOH was added to the substance and the mixture was reacted at 5 to 10° C for an hour to split the protective group off. After completion of the reaction, precipitation was preformed with anhydrous ether to give 7.9 g of the product (yield, 89%).
Melting point: 128° C (decomposed)

| Elemental analysis: $C_{14}H_{21}N_2O_2Cl.2/5H_2O$ | | | |
|---|---|---|---|
| | C (%) | H (%) | N (%) |
| Calcd. | 57.59 | 7.52 | 9.59 |
| Found | 57.44 | 7.60 | 9.44 |

Example 2

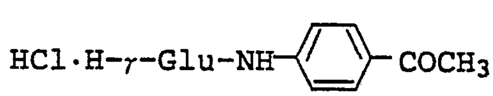

Using 1.2 g of BOC-Glu-OBut, 0.5 g of p-aminoacetophenone, 0.8 g of WSCD and 10 ml of pyridine, the reaction and post-treatment were carried out in a manner similar to Example 1. Then, the protective group was removed with 2N-HCl/AcOH followed by gel filtration using TOYOPEARL HW40F (30% AcOH). The fraction containing the product was collected and freeze dried to give 0.6 g of the product (yield, 71%).

| Elemental analysis: $C_{13}H_{17}N_2O_4Cl$ | | | |
|---|---|---|---|
| | C (%) | H (%) | N (%) |
| Calcd. | 51.92 | 5.70 | 9.31 |
| Found | 52.22 | 5.87 | 9.47 |

Example 3

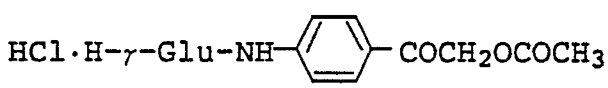

Using 1.36 g of BOC-Glu-OBut, 0.86 g of p-amino-ω-oxyacetophenone, 0.50 g of HOBt, 0.09 g of WSCD and 20 ml of DMF, the reaction and post-treatment were carried out in a manner similar to Example 1. The resulting crude product was recrystallized from AcOET/n-Hex to give 1.61 g (yield, 75%) of:

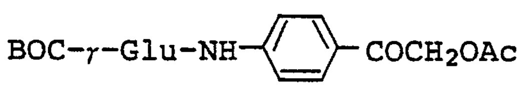

In a moistureproof state, 6.2 ml of 2N-HCl/AcOH was added to 0.6 g of the above product and the mixture was reacted at room temperature for an hour. Anhydrous ether was added to the reaction mixture and the resulting precipitates were collected by filtration and dried in vacuum to give 0.41 g of the product. Melting point: 179-184 °C

| Elemental analysis: $C_{15}H_{19}N_2O_5Cl.1/2H_2O$ | | | |
|---|---|---|---|
| | C (%) | H (%) | N (%) |
| Calcd. | 48.99 | 5.76 | 7.62 |
| Found | 48.90 | 5.41 | 7.41 |

Example 4

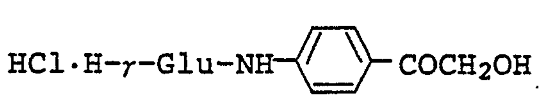

After 0.96 g of:

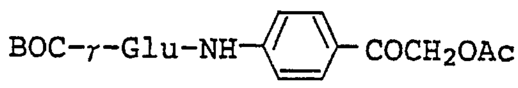

obtained in Example 3 was dissolved in 10 ml of anhydrous methanol, 0.1 ml of 28% $CH_3ONa$-MeOH was added to the solution. The mixture was stirred at 5° C overnight to react them. After completion of the reaction, 100 ml of AcOEt was added to the reaction mixture followed by washing with saturated sodium chloride aqueous solution, cold 5% HCl, saturated $NaHCO_3$ aqueous solution and then saturated sodium chloride aqueous solution. The organic phase was then dried over anhydrous magnesium sulfate. The solvent was distilled off in vacuum to give the crude product. The crude product was recrystallized from AcOEt/n Hex to give 0.56 g of:

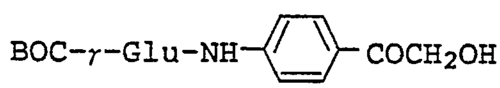

(yield, 64%). Melting point, 129 - 136° C.

In a moistureproof state, 5 ml of 2N-HCl/AcOH was added to the above product and the mixture was reacted at room temperature for an hour. Then anhydrous ether was added to the mixture. The resulting precipitates were collected by filtration and dried in vacuum to give 0.2 g of the product (yield, 50%).
Melting point: 84-87° C

| Elemental analysis: $C_{13}H_{17}N_2O_5Cl.2/5H_2O$ | | | |
|---|---|---|---|
| | C (%) | H (%) | N (%) |
| Calcd. | 48.20 | 5.54 | 8.65 |
| Found | 48.34 | 5.58 | 8.33 |

Example 5

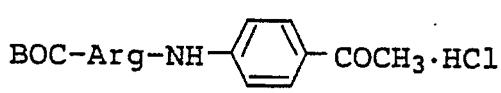

Using 8.2 g of BOC-Arg-OH•HCl•$H_2O$, 3.8 g of p-aminoacetophenone, 9.6 g of WSCD and 70 ml of pyridine, the reaction was carried out in a manner similar to Example 1. Then, the reaction mixture was extracted with AcOEt/n-BuOH (75 ml : 40 ml). After the extract was washed with saturated sodium chloride aqueous solution, cold 5% HCl, saturated $NaHCO_3$ aqueous solution and then saturated sodium chloride aqueous solution, the organic phase was dried over anhydrous magnesium sulfate. The solvent was distilled off in vacuum to give 8.8 g of the product as foam (yield, 79%).

| Elemental analysis: $C_{19}H_{30}N_5OCl.1/5H_2O$ | | | |
|---|---|---|---|
| | C (%) | H (%) | N (%) |
| Calcd. | 52.88 | 7.10 | 16.23 |
| Found | 52.87 | 7.32 | 15.90 |

Example 6

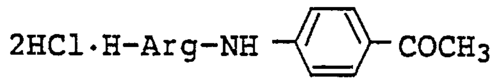

After 47.5 ml of 2N-HCl/AcOH was added to 8.8 g of:

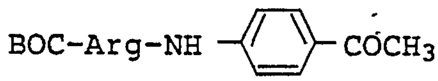

obtained in Example 5, the mixture was reacted at 5° C for 2 hours. The reaction mixture was added to anhydrous ether to precipitate. Thus 6.9 g of the product was obtained in an amorphous state (yield, 100%).

| Elemental analysis: $C_{14}H_{23}N_5O_2Cl_2.1/10H_2O$ | | | |
|---|---|---|---|
| | C (%) | H (%) | N (%) |
| Calcd. | 46.16 | 6.36 | 19.23 |
| Found | 45.93 | 6.38 | 19.13 |

Example 7

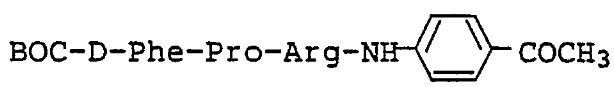

After 6.74 g of:

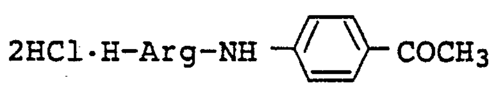

obtained in Example 6, 6.70 g of BOC-D-Phe-Pro-OH and 2.83 g of HOBt were dissolved in 155 ml of DMF. The solution was then cooled to 5° C and 18.5 ml of NEM was added to the solution and 3.89 g of WSCD was further added to the mixture. After reacting at 5° C for 4 hours, the mixture was further reacted at room temperature overnight. After completion of the reaction, DMF was distilled off in vacuum and the residue was dissolved in MeOH/AcOEt (22 ml : 190 ml) followed by washing with saturated sodium chloride aqueous solution, cold 5% HCl, saturated $NaHCO_3$ aqueous solution and then saturated sodium chloride aqueous solution. The organic phase was then dried over anhydrous magnesium sulfate. The solvent was distilled off in vacuum to give the oily product. The oily product was applied to Sephadex LH-20 (MeOH) for gel filtration. The fraction containing the product was collected, concentrated and recrystallized from AcOEt/n-Hex to give 9.4 g of the product (yield, 59%).
Melting point: 97-116° C

| Elemental analysis: $C_{33}H_{46}N_7O_6Cl.1/2MeOH$ | | | |
|---|---|---|---|
| | C (%) | H (%) | N (%) |
| Calcd. | 58.46 | 7.03 | 14.25 |
| Found | 58.52 | 7.19 | 13.96 |

Example 8

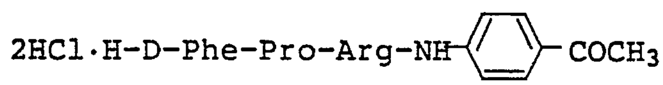

After 26.3 ml of 2N-HCl/AcOH was added to 7.06 g of the protected tripeptide obtained in Example 7, the reaction was carried out at room temperature for an hour in a moistureproof state. After completion of the reaction, anhydrous ether was added and the formed precipitates were collected by filtration followed by gel filtration using TOYOPEARL HW40F (30% AcOH). The fraction containing the product was collected and freeze dried to give 5.26 g of the product (yield, 82%).

| Elemental analysis: $C_{28}H_{39}N_7O_4Cl_2.2H_2O$ | | | |
|---|---|---|---|
| | C (%) | H (%) | N (%) |
| Calcd. | 52.17 | 6.72 | 15.21 |
| Found | 52.24 | 6.52 | 15.07 |

Example 9

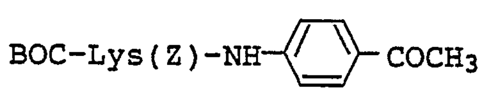

Using 38.0 g of BOC-Lys(Z)-OH, 13.5 g of p-aminoacetophenone, 21.0 g of WSCD and 200 ml of pyridine, the reaction and post-treatment were carried out in a manner similar to Example 1. Then, recrystallization was performed from AcOEt/n-Hex to give 43.8 g of the product (yield, 88%).
Melting point: 110-119 °C

| Elemental analysis: $C_{27}H_{35}N_3O_6$ | | | |
|---|---|---|---|
| | C (%) | H (%) | N (%) |
| Calcd. | 65.17 | 7.09 | 8.45 |
| Found | 65.12 | 7.22 | 8.45 |

Example 10

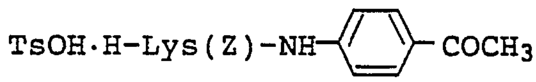

After 25 g of:

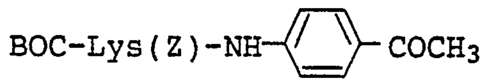

obtained in Example 9 was dissolved in 140 ml of AcOEt, the solution was heated to 30° C and a solution of 35 g of TsOH in 90 ml of AcOEt was added to the solution. The mixture was reacted for 3 hours while stirring. After completion of the reaction, the precipitated crystals were collected by filtration and dried in vacuum to give 20.1 g of the product (yield, 71%).
Melting point: 174-183° C

| Elemental analysis: $C_{29}H_{36}N_3O_7S \cdot 1/2H_2$ | | | |
|---|---|---|---|
| | C (%) | H (%) | N (%) |
| Calcd. | 60.09 | 6.40 | 7.25 |
| Found | 60.17 | 6.20 | 6.99 |

Example 11

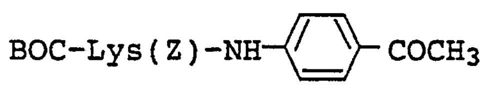

After 7.41 g of:

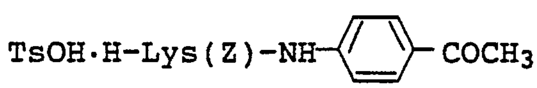

obtained in Example 10 was dissolved in 26 ml of DMF, the solution was cooled to 5° C and 5.04 g of BOC-Phe-SDP powders were added to the solution. The mixture was reacted for 4 hours at 5° C and then at room temperature overnight. After completion of the reaction, DMF was distilled off in vacuum and the residue was dissolved in 190 ml of AcOEt. After washing with saturated sodium chloride aqueous solution, cold 5% HCl, saturated $NaHCO_3$ aqueous solution and then saturated sodium chloride aqueous solution, the organic phase was dried over anhydrous magnesium sulfate. The solvent was distilled off in vacuum to give 7.48 g of the product as crystals (yield, 90%).
Melting point: 167-172° C

| Elemental analysis: $C_{36}H_{44}N_4O_7$ | | | |
|---|---|---|---|
| | C (%) | H (%) | N (%) |
| Calcd. | 66.96 | 7.02 | 8.67 |
| Found | 66.81 | 7.13 | 8.59 |

Example 12

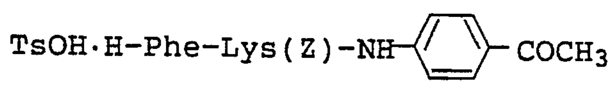

After 7.41 g of:

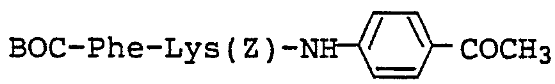

obtained in Example 11 was dissolved in 34 ml of AcOEt, a solution of 8.75 g of TsOH in 23 ml of AcOEt was added to the solution. The mixture was reacted for 3 hours at 30 °C. Then 60 ml of ether was added to the reaction mixture to precipitate crystals. The crystals were collected by filtration and dried in vacuum to give 7.48 g of the product (yield, 90%).

Melting point: 189-193 °C

| Elemental analysis: $C_{38}H_{44}N_4O_8S$ | | | |
|---|---|---|---|
| | C (%) | H (%) | N (%) |
| Calcd. | 63.67 | 6.19 | 7.82 |
| Found | 63.64 | 6.37 | 7.62 |

Example 13

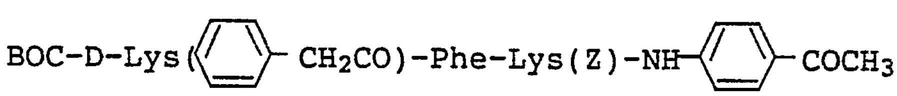

After 3.58 g of:

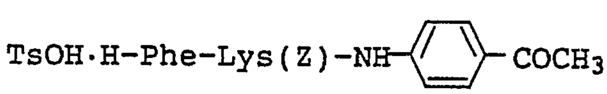

obtained in Example 12 was dissolved in 40 ml of DMF, the solution was cooled to 5 °C and 0.65 ml of NEM was added to the solution and 2.31 g of

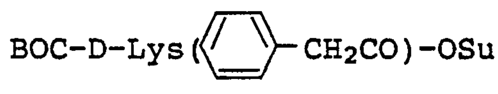

powders were then added to the mixture. After reacting at 5° C for 4 hours, the mixture was further reacted at room temperature overnight. After completion of the reaction, DMF was distilled off in vacuum and the residue was dissolved in 200 ml of AcOEt followed by washing with saturated sodium chloride aqueous solution, cold 5% HCl, saturated $NaHCO_3$ aqueous solution and then saturated sodium chloride aqueous solution. The organic phase was then dried over anhydrous magnesium sulfate. The solvent was distilled off in vacuum to give 3.83 g of the product as crystals (yield, 85%).
Melting point: 145-147° C

| Elemental analysis: $C_{50}H_{62}N_6O_9.3/5H_2O$ | | | |
|---|---|---|---|
| | C (%) | H (%) | N (%) |
| Calcd. | 66.59 | 7.06 | 9.32 |
| Found | 66.51 | 7.06 | 9.54 |

Example 14

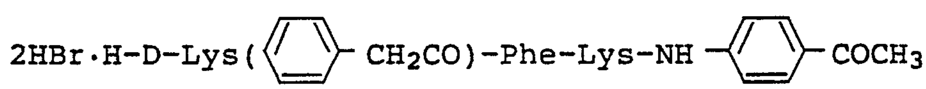

After 27.2 ml of 25% HBr/AcOH was added to 3.24 g of the protected tripeptide obtained in Example 13 in a moistureproof state, the reaction was carried out at room temperature for an hour while stirring. Then the reaction mixture was added to 500 ml of anhydrous ether and the formed precipitates were collected by filtration followed by gel filtration using TOYOPEARL HW40F (30% AcOH). The fraction containing the product was collected and freeze dried to give 1.94 g of the product (yield, 64%).

| Elemental analysis: $C_{37}H_{50}N_6O_5Br_2.3/2H_2O$ | | | |
|---|---|---|---|
| | C (%) | H (%) | N (%) |
| Calcd. | 52.55 | 6.32 | 9.94 |
| Found | 52.53 | 6.17 | 9.84 |

Example 15 Determination of leucine aminopeptidase (LAP) activity in serum

(1) 125 mM Tris buffer, pH 7.8 (25° C)
(2) sample
(3) 20.4 mM substrate (Example 1) solution

After 0.05 ml of the sample was added to 2.0 ml of the buffer (1), the mixture was previously heated to 37° C for about 2 to about 10 minutes and 0.5 ml of the substrate solution (3) was added to the solution. At the same time a stop watch was started. Accurately 30 seconds and 90 seconds after, absorbance was measured at 340 nm to determine a change in absorbance for one minute. Time course of the reaction is shown in Fig. 2. As the sample, human serum was used. The leucine aminopeptidase activity is calculated

in accordance with the following equation:

$$I\ U/\ell = \frac{\Delta O.D/min^{1)} \times \text{total volume of the reaction solution } 10^6}{\text{molecular extinction coefficient}^{2)} \times \text{volume of serum}}$$

1) ΔO.D/min indicates a change in absorbance per one minute at a measurement wavelength of 340 nm.

2) The molecular extinction coefficient at a wavelength of 340 nm is 6284.

According to the above equation, the activity of the serum used was found to be 135 (IU/ℓ) units.

As shown in Fig. 2, the results are shown by a straight line for 10 minutes without any time lag with passage of time, indicating that the results are usable for an automated analysis equipment.

Example 16 Determination of γ-glutamyltranspeptidase (γ-GTP) activity in serum

(1) 200 mM Tris-100 mM glycylglycine buffer, pH 8.3 (25° C)
(2) sample
(3) 50 mM substrate (Example 2) solution

After 0.2 ml of the sample was added to 4.0 ml of the buffer (1), the mixture was previously heated to 37° C for about 2 to about 10 minutes and 0.4 ml of the substrate solution (3) was added to the solution. At the same time, a stop watch was started. Accurately 30 seconds and 90 seconds after, absorbance was measured at 340 nm to determine a change in absorbance for one minute. Time course of the reaction is shown in Fig. 3.

As the sample, human serum was used. The γ-glutamyltranspeptidase activity in serum is calculated in accordance with an equation similar to Example 13.

Also in the measurement using the compounds obtained in Examples 3 and 4 as substrate, the activity is determined in a manner similar to that described above using the reagents described above. Time course of each reaction is shown in Figs. 4 and 5, respectively.

Example 17 Standard curve of p-aminoacetophenone

(1) Buffer of Example 15
(2) 0.1 mM to 0.5 mM of p-aminoacetophenone (final concentration of 20 µM to 100 µM)

Accurately 5 ml of the aqueous solution (2) in each concentration was weighed in a measuring flask of 20 ml and further accurately regulated with the buffer (1). After each solution was previously heated for about 2 to about 10 minutes, absorbance in each concentration was measured at 340 nm. Fig. 6 shows the standard curve.

As is shown in Fig. 6, the results show a straight line crossing the origin up to the concentration of 100 µM. This indicates that the results are sufficiently quantitative.

Example 18 Determination of antithrombin-III (AT-III)

(1) 75 mM Tris buffer containing 0.5 NIH/ml of thrombin (TH) and 0.5 U/ml of heparin, pH 8.6 (25° C)
(2) 10 mM substrate (Example 8) aqueous solution
(3) human plasma (diluted to 5-fold with physiological saline)

After 0.025 ml of the sample (3) was added to 2.0 ml of the buffer (1), the mixture was previously heated to 37° C for about 2 to about 10 minutes and 0.5 ml of the substrate solution (3) was added to the solution. At the same time, a stop watch was started. Accurately 30 seconds and 90 seconds after, absorbance was measured at 340 nm to determine a change in absorbance for one minute. Change in absorbance per one minute was plotted on the dilution magnification of the sample to prepare a calibration curve. The results are shown in Fig. 7.

The results reveal that AT-III can be determined in high sensitivity with good quantitative property, using

the novel substrate of the present invention.

Example 19 Determination of plasminogen

(1) 150 mM Tris-maleate buffer containing 0.27 U/ml of streptokinase (SK), pH 6.8 (25° C)
(2) 10 µM substrate (Example 14) aqueous solution
(3) human plasma (diluted to 5-fold with physiological saline)

After 0.2 ml of the sample (3) was added to 3.0 ml of the buffer (1), the mixture was previously heated to 37° C for about 2 to about 10 minutes and 0.5 ml of the substrate solution (3) was added to the solution. At the same time, a stop watch was started. Accurately 30 seconds and 90 seconds after, absorbance was measured at 340 nm to determine a change in absorbance for one minute. Change in absorbance per minute was plotted on the dilution magnification of the sample to prepare a calibration curve. The results are shown in Fig. 8.

The results reveal that PLG can be determined in high sensitivity with good quantitative property, using the novel substrate of the present invention.

**Claims**

1. An aminoacetophenone derivative represented by general formula (I):

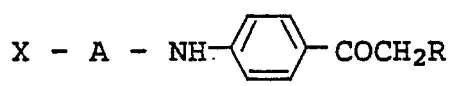

(I)

wherein R represents hydrogen, an alkyl group, hydroxy group or acetoxy group; X represents hydrogen atom, a group for irreversibly masking the terminal amino group or a amino-protective group conventionally used in peptide chemistry; A represents an L- or D-amino acid residue selected from the group consisting of phenylalanine, leucine, isoleucine, glutamic acid, pyroglutamic acid, serine, valine, arginine, lysine, proline, pipecolic acid, alanine and phenylalanine or a homologue thereof, or a residue of di- or tripeptide composed of 2 or 3 of these amino acids or a homologue thereof; provided that ε-amino group of the lysine residue may optionally be protected with a protective group; and its acid addition salt.

2. An aminoacetophenone derivative of claim 1, wherein said alkyl group has 1 to 5 carbon atoms.

3. An aminoacetophenone derivative of claim 2, wherein said alkyl group is selected from the group consisting of methyl, ethyl, propyl, butyl and pentyl.

4. An aminoacetophenone derivative of claim 1, wherein said masking group is benzoyl.

5. An aminoacetophenone derivative of claim 1, which is leucyl-4-acetylanilide.

6. An aminoacetophenone derivative of claim 1, which is γ-glutamyl-4-acetylanilide.

7. An aminoacetophenone derivative of claim 1, which is γ-glutamyl-4-(γ-acetoxyacetyl)anilide.

8. An aminoacetophenone derivative of claim 1, which is γ-glutamyl-4-(γ-oxyacetyl)anilide.

9. An aminoacetophenone derivative of claim 1, which is any one of compounds of tripeptides shown below:

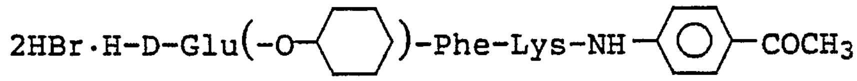

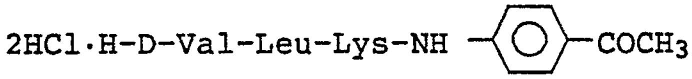

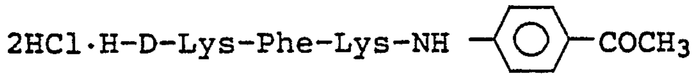

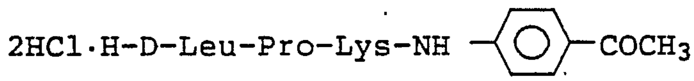

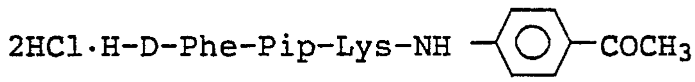

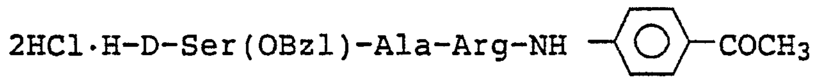

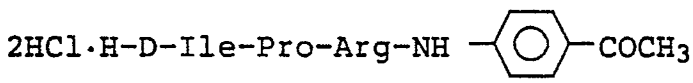

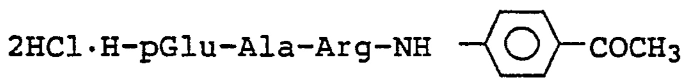

10. A method for determination of a peptidase activity or a protease activity which comprises using an aminoacetophenone derivative represented by general formula (I):

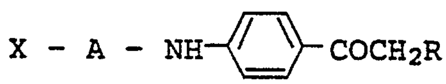 (I)

wherein R represents hydrogen, an alkyl group, hydroxy group or acetoxy group; X represents hydrogen atom, a group for irreversibly masking the terminal amino group or a amino-protective group conventionally used in peptide chemistry; A represents an L- or D-amino acid residue selected from the group consisting of phenylalanine, leucine, isoleucine, glutamic acid, pyroglutamic acid, serine, valine, arginine, lysine, proline, pipecolic acid, alanine and phenylalanine or a homologue thereof, or a residue of di- or tripeptide composed of 2 or 3 of these amino acids or a homologue thereof; provided that ε-amino group of the lysine residue may optionally be protected with a protective group.

11. A method for determination of a peptidase activity or a protease activity in a serum or plasma sample which comprises mixing said sample with an aminoacetophenone derivative represented by general formula (I):

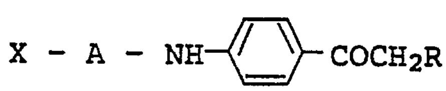 (I)

wherein R represents hydrogen, an alkyl group, hydroxy group or acetoxy group; X represents hydrogen atom, a group for irreversibly masking the terminal amino group or a amino-protective group conventionally

used in peptide chemistry; A represents an L- or D-amino acid residue selected from the group consisting of phenylalanine, leucine, isoleucine, glutamic acid, pyroglutamic acid, serine, valine, arginine, lysine, proline, pipecolic acid, alanine and phenylalanine or a homologue thereof, or a residue of di- or tripeptide composed of 2 or 3 of these amino acids or a homologue thereof; provided that ε-amino group of the lysine residue may optionally be protected with a protective group; or its acid addition salt and measuring absorbance of the resulting compound represented by general formula (II):

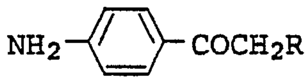

(II)

wherein R has the same significance as described above.

12. A method according to claim 10 or 11, wherein said absorbance of the compound represented by general formula (II) is measured at a wavelength between about 335 and about 355 nm.

FIG. I

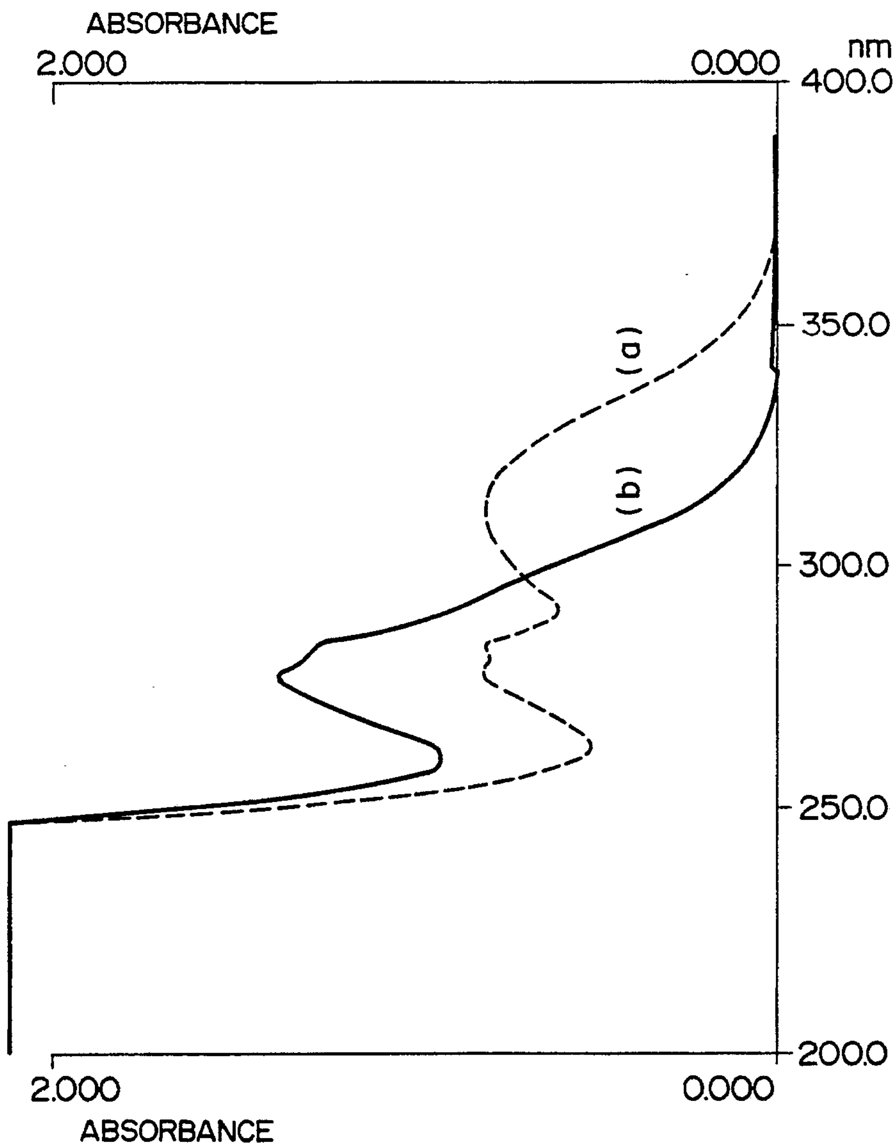
ABSORBANCE
2.000
0.000
nm
400.0
350.0
300.0
250.0
200.0
(a)
(b)
2.000
0.000
ABSORBANCE

FIG. 2

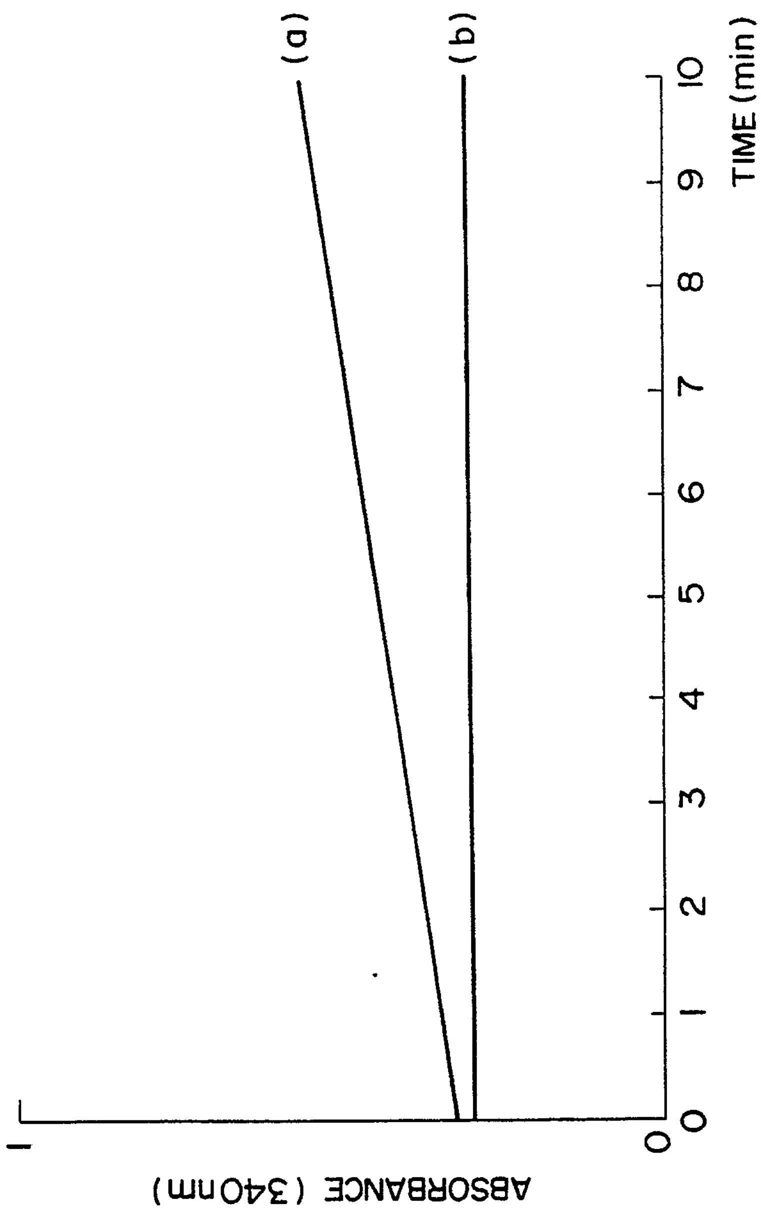

FIG. 3

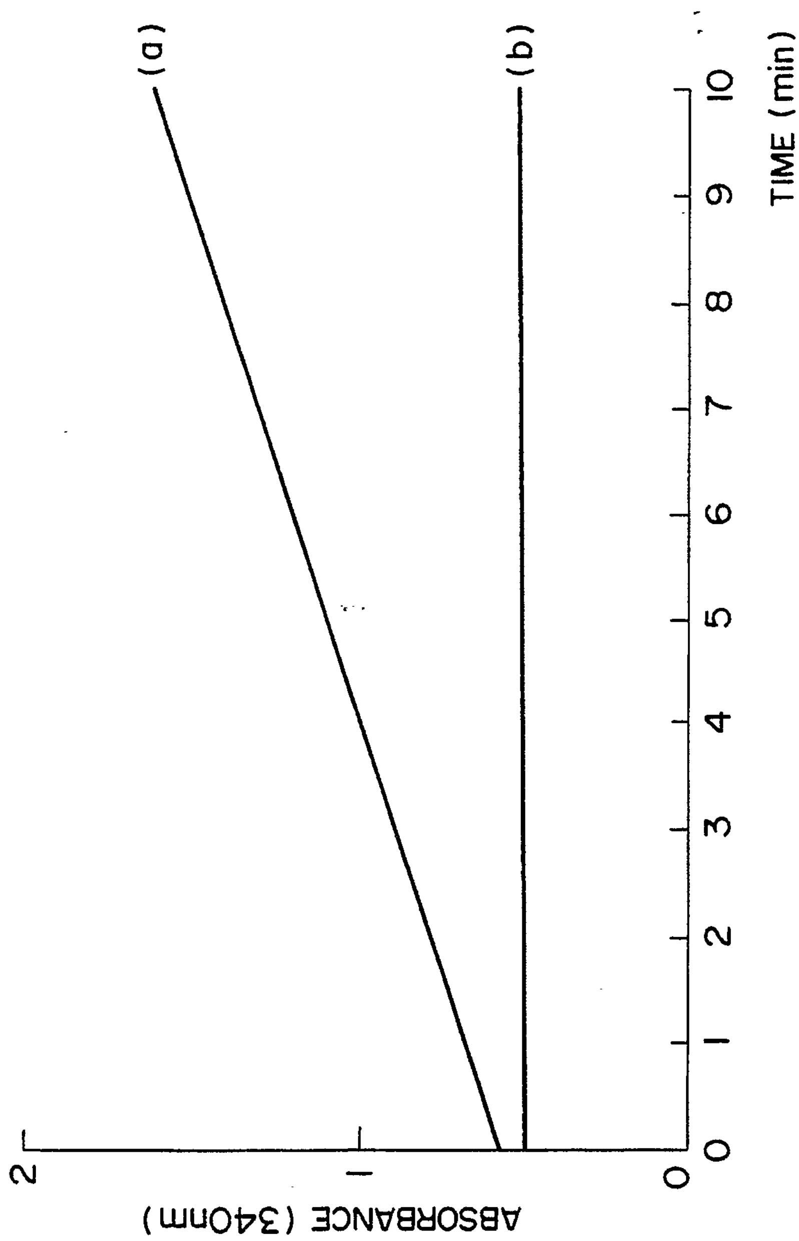

FIG. 4

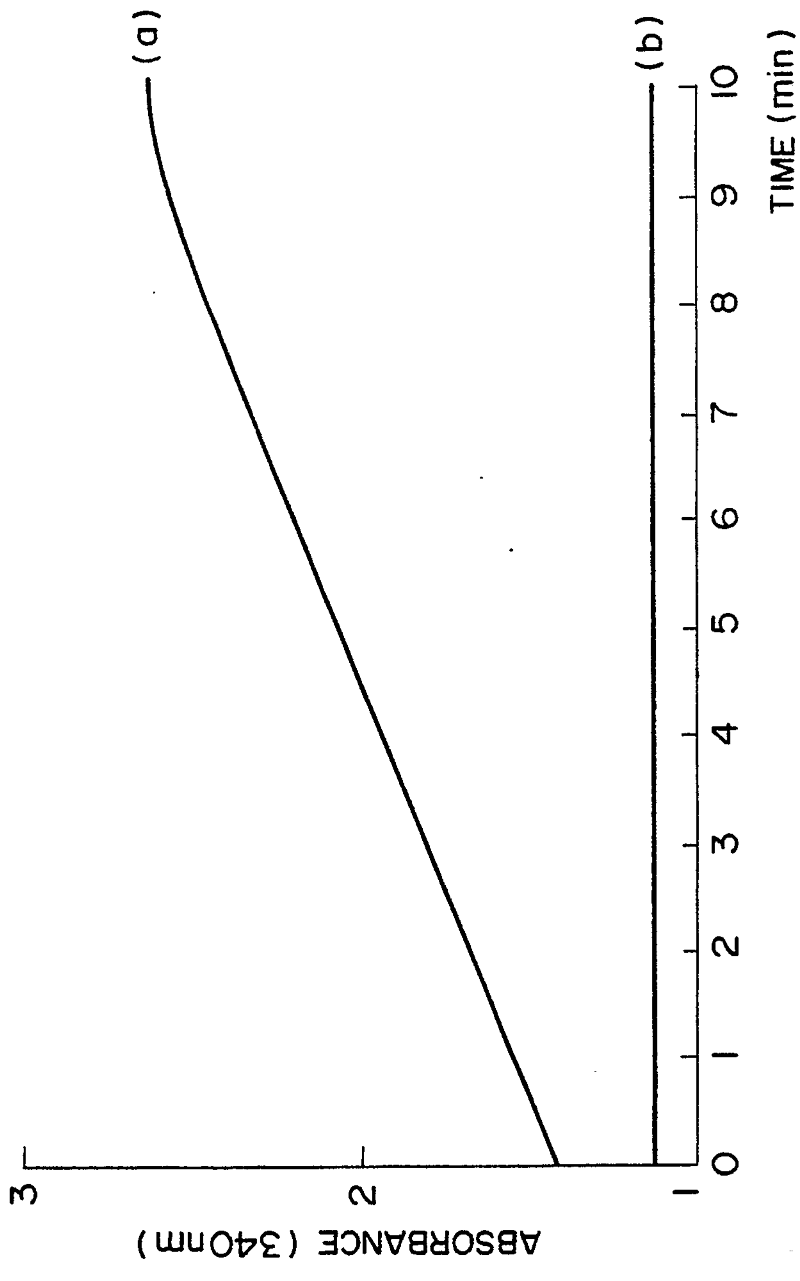

FIG. 5

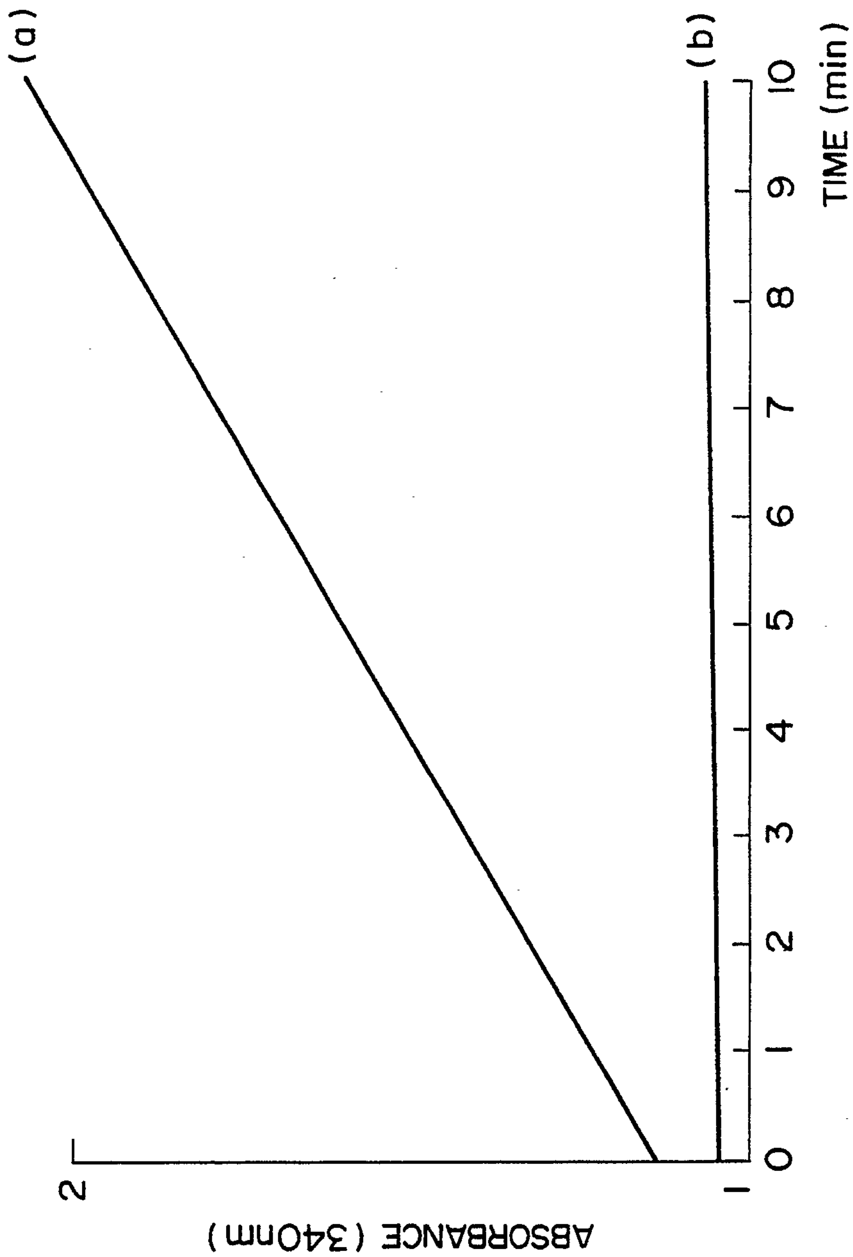

FIG. 6

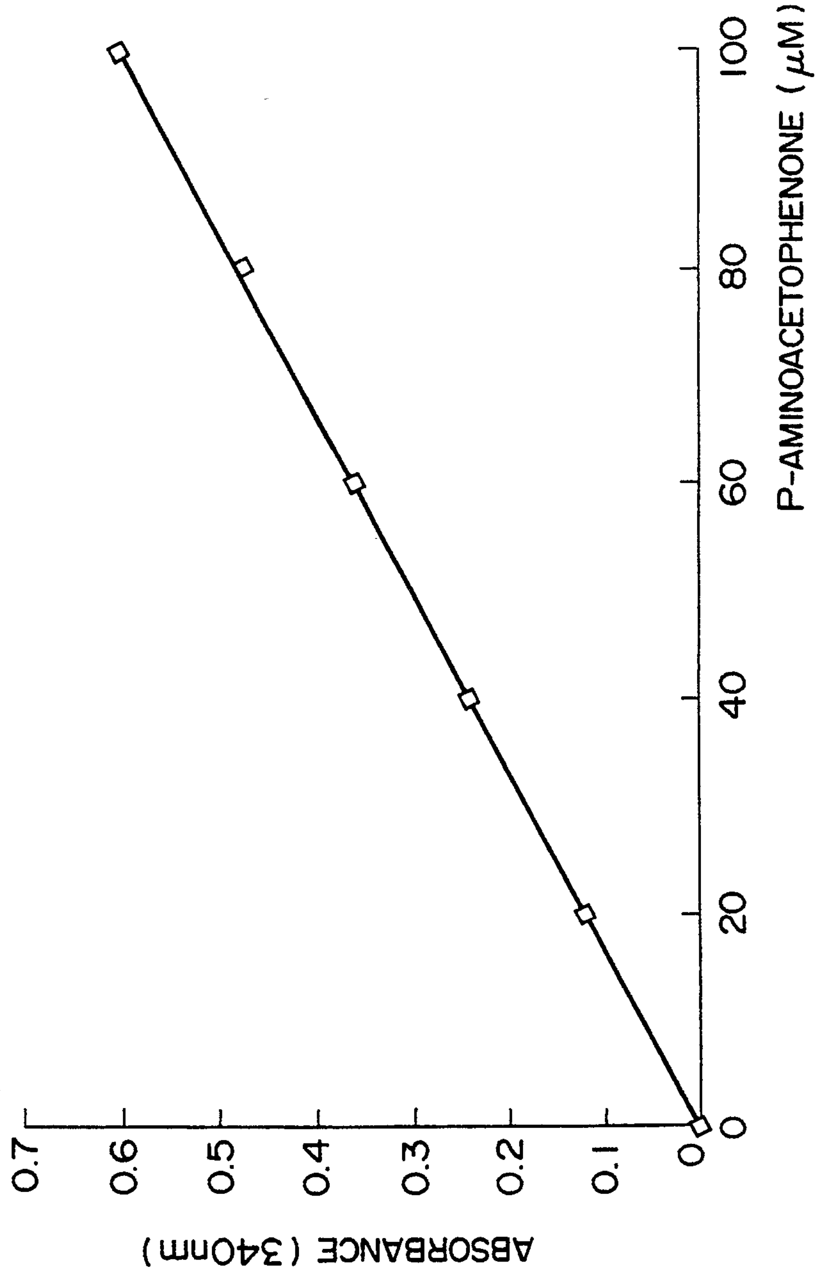
ABSORBANCE ( 340nm )
0
0.1
0.2
0.3
0.4
0.5
0.6
0.7
0
20
40
60
80
100
P-AMINOACETOPHENONE ( μM )

FIG. 7

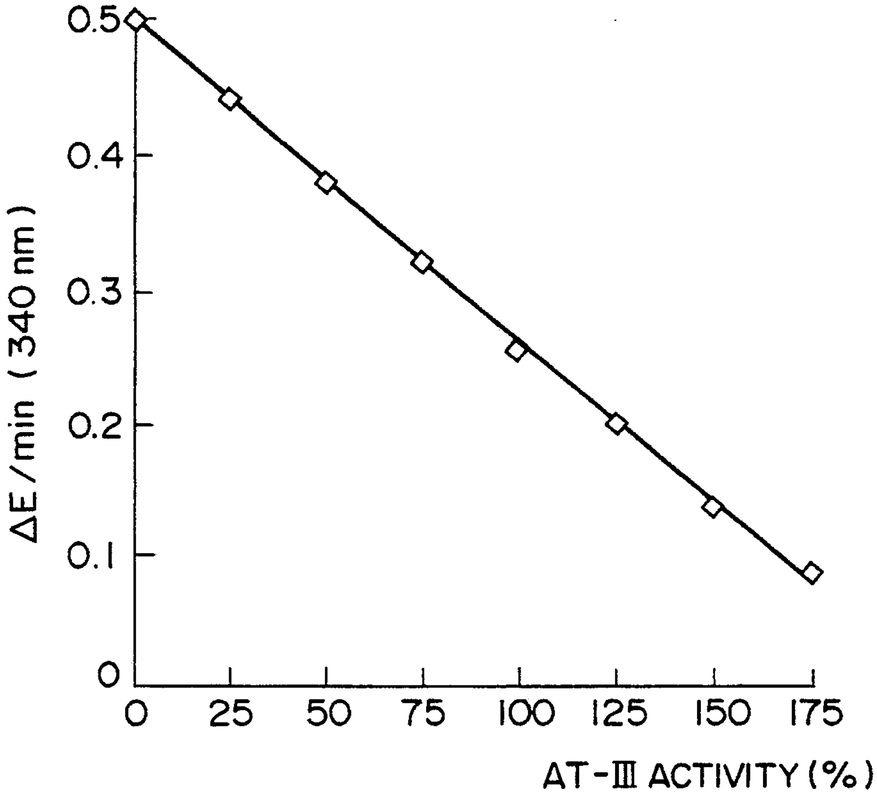
0.5
0.4
0.3
0.2
0.1
0
ΔE / min ( 340 nm )
0
25
50
75
100
125
150
175
AT-III ACTIVITY (%)

FIG. 8

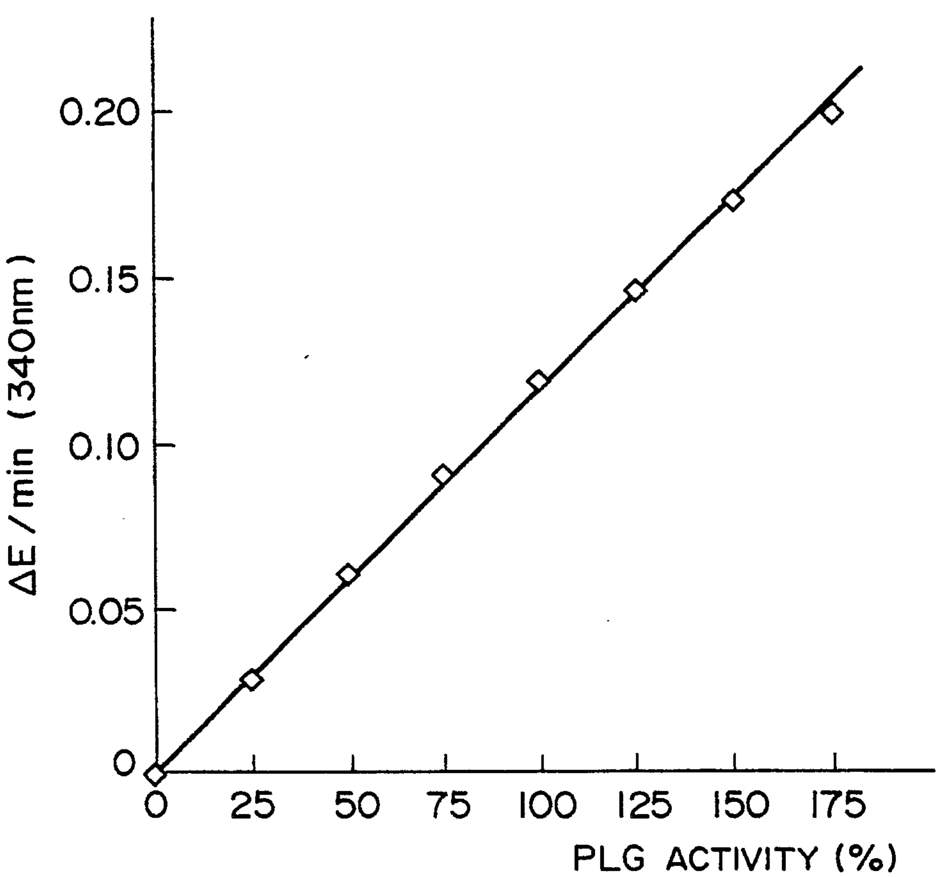
0.20
0.15
0.10
0.05
0
ΔE / min (340nm)
0
25
50
75
100
125
150
175
PLG ACTIVITY (%)